# EUROPEAN PATENT SPECIFICATION

(11) **EP 3 422 869 B1**
(45) Date of publication and mention of the grant of the patent: **13.03.2024**
(21) Application number: 17760593.8
(22) Date of filing: 28.02.2017
(51) Int. Cl.: A23L 2/52, A23L 27/00, C12C 5/02, C12G 3/04, A23L 2/56, A23L 27/20, C12G 3/06, A61K 31/56, A23L 33/11

(54) **NOVEL COMPOSITIONS FOR FLAVOR ENHANCEMENT**
NEUARTIGE ZUSAMMENSETZUNGEN ZUR GESCHMACKSVERBESSERUNG
NOUVELLES COMPOSITIONS DE RENFORCEMENT DU GOÛT

(30) Priority: 02.03.2016 US 201662302418 P; 19.04.2016 US 201662324385 P
(43) Date of publication of application: 09.01.2019
(73) Proprietor: International Flavors & Fragrances Inc., New York, NY 10019 (US)
(72) Inventor: WU, Hou, East Brunswick New Jersey 08816 (US); JOHN, Thumpalasseril V., Morganville New Jersey 07751 (US); KIM, Jung-A., Edgewater New Jersey 07020 (US); MURANO, Kathryn, Staten Island New York 10314 (US); WANSOR, Robert, Newtown Pennsylvania 18940 (US)
(74) Representative: Barker Brettell LLP
(86) International application number: PCT/US2017/019903
(87) International publication number: WO 2017/151589

(56) References cited:
- EP-A1- 1 959 763
- WO-A1-02/28204
- WO-A1-99/15547
- WO-A1-99/63841
- WO-A1-2005/049037
- WO-A2-00/41491
- KR-A- 20080 112 239
- US-A1- 2004 029 844
- US-A1- 2005 064 078
- US-A1- 2007 026 119
- US-A1- 2007 116 824
- US-A1- 2011 117 262
- US-B1- 6 441 206
- DATABASE GNPD [Online] MINTEL; 29 April 2002 (2002-04-29), anonymous: "Orange Sports Drink", XP055630046, retrieved from www.gnpd.com Database accession no. 10107255
- LACHENMEIER, DIRK W. ET AL.: 'Alcoholic beverage strength discrimination by taste may have an upper threshold' ALCOHOLISM: CLINICAL AND EXPERIMENTAL RESEARCH vol. 38, no. 9, September 2014, pages 2460 - 2467, XP055414956

## Description

### Field of the Invention

The present invention relates to methods for enhancing alcohol sensation in alcoholic consumables or alcohol-free beverages. The present invention further relates to methods for enhancing carbonation effect in carbonated beverages.

### Background of the Invention

There is an ongoing need in the flavor industry for taste modifying compounds that improve, enhance or modify flavors for food preparations. Those with skill in the art appreciate how differences in the chemical structures of the molecules can result in significant differences in functions. The identification of structural variations and discovery of new compounds enable the creation of new flavors.

Phytosterols occur in plants and encompass sterols and stands. Stands are saturated forms of corresponding sterols. Phytosterols are steroid compounds similar to cholesterol. They are poorly absorbed and can compete with cholesterol for absorption in the intestine, resulting lower levels of cholesterol. Phytosterol-enriched foods and dietary supplements have been marketed for decades. Phytosterols are also disclosed as functional ingredients to supplement food and beverages especially sweetener compositions to promote health and wellness (U. S. Patent No.s 6,129,944 and 9,131,720). A naturally occurring phytosterol blend CHOLESTATIN^{®} is reported to provide enhanced flavorings such as vanilla, chocolate, butter, cheese, strawberry, raspberry, blueberry, orange, lemon, apple, grape, lemon-lime, lime, watermelon, coconut, beef, bacon, chicken, pork, onion, garlic, pepper, ranch, nacho, taco, cheddar, romano, parmesan, cream, buttermilk, blue cheese and combinations thereof, and is therefore proposed to be incorporated in a wide variety of foods including popcorn, baked goods, cheese sauce, dips, condiments, dressings, marinades, fillings, toppings, snack blends and side dishes, cereals, yogurt, fried foods, prepared meals, dairy products, frostings, gravies, ice cream, snacks and chips, crackers, puddings, candies and nutritional bars (U. S. Publication No. 2005/0064078).

More than 200 sterols and related compounds have been identified (Akhisa, et al. (1991) In: Physiology and Biochemistry of Sterols, Patterson, G.W. and W.D. Nes (Eds.). American Oil Chemists Society, Champaign, IL, USA 172-228). However, available sterols exist mostly as mixtures and the reported separations are laborious (Zhang, et al. (2005) Steroids, 70(13): 886-895). In the flavor industry, none of the individual sterols has been isolated, investigated and conclusively characterized.

WO 99/15547 A1 disclosed beverage compositions and acetic acid compositions that contain hydroxy acid, keto acid, dicarboxylic acid or amino acid esters of a phytosterol and/or a phytostanol, or their salts, the amine salts of amino acid esters, polyol esters, poly- or oligoesters, or amide or alpha -amino acid amide derivatives thereof.

WO 2005/049037 A1 disclosed a process for producing a substantially stable dispersion without manufacturing aids, where the dispersion comprises at least one hydrophobic plant sterol and an aqueous material, wherein the plant sterol is selected from plant sterols and plant stanols.

WO 99/63841 A1 disclosed a composition suitable for incorporation into foods, beverages, pharmaceuticals, nutraceuticals and the like comprises one or more phytosterols, phytostanols or mixtures of both, treated to enhance the solubility and dispersability thereof.

KR 20080112239 A disclosed a plant sterol-containing milk beverage in which a plant sterol can be emulsified stably for a prolonged storage period and is excellent in quality-retaining property and stability.

WO 00/41491 A2 disclosed a method for making a composition suitable for inclusion in a food product or beverage the method comprising the step of combining a hydrophobic compound which can be shown to be beneficial for human health with a component which is acceptable as a food additive, wherein the component which is acceptable as a food additive interacts with the surface of the hydrophobic compound.

It has now been found unexpectedly that sitosterol, 17-(4-ethyl-1,5-dimethyl-hexyl)-10,13-dimethyl-2,3,4,7,8,9,10,11,12,13,14,15,16,17-tetradecahydro-1H-cyclopenta[a]phenanthren-3-ol (Formula I), and in particular an isomer, β-sitosterol, (3S,8S,9S,10R,13R,14S,17R)-17-((1R,4R)-4-ethyl-1,5-dimethyl-hexyl)-10,13-dimethyl-2,3,4,7,8,9,10,11,12,13,14,15,16,17-tetradecahydro-1H-cyclopenta[a]phenanthren-3-ol (Formula II), surprisingly enhances alcohol sensation in alcoholic consumables or alcohol-free beverages. Sitosterol such as β-sitosterol has also been found unexpectedly to enhance carbonation effect in carbonated beverages.

### Summary of the Invention

The scope of the invention is defined in the claims.

The present invention provides a method of enhancing alcohol sensation in an alcoholic consumable or an alcohol-free beverage comprising the step of adding an olfactory effective amount of 17-(4-ethyl-1,5-dimethyl-hexyl)-10,13-dimethyl-2,3,4,7,8,9,10,11,12,13,14,15,16,17-tetradecahydro-1H-cyclopenta[a]phenanthren-3-ol or an isomer thereof, wherein the olfactory effective amount is 0.1 parts per billion or greater by weight.

The invention further provides a method of enhancing carbonation effect in a carbonated beverage comprising the step of adding an olfactory effective amount of 17-(4-ethyl-1,5-dimethyl-hexyl)-10,13-dimethyl-2,3,4,7,8,9,10,11,12,13,14,15,16,17-tetradecahydro-1H-cyclopenta[a]phenanthren-3-ol or an isomer thereof, wherein the olfactory effective amount is 0.1 parts per trillion or greater by weight.

### Sitosterol, 17-(4-ethyl-1,5-dimethyl-hexyl)-10,13-dimethyl-2,3,4,7,8,9,10,11,12,13,14,15,16,17-tetradecahydro-1H-cyclopenta[a]phenanthren-3-ol (Formula I), is represented by the formula set forth below:

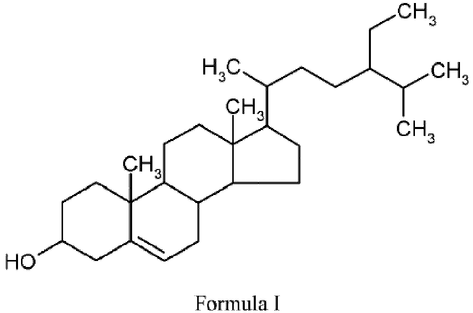

### Detailed Description of the Invention

Among the numerous sterols, some of the most commonly known ones include, for example, sitosterol, campesterol, stigmasterol, brassicasterol and ergosterol. Sitosterol further includes isomers such as a-sitosterol, β-sitosterol and γ-sitosterol. β-Sitosterol is the most common isomeric form of sitosterol. The close structures of some of the sterols and cholesterol are set forth in the following. Those with skill in the art appreciate how small structural differences can result in unexpected and significant differences in properties and functions.

### Sitosterol, 17-(4-ethyl-1,5-dimethyl-hexyl)-10,13-dimethyl-2,3,4,7,8,9,10,11,12,13,14, 15,16,17-tetradecahydro-1H-cyclopenta[a]phenanthren-3-ol:

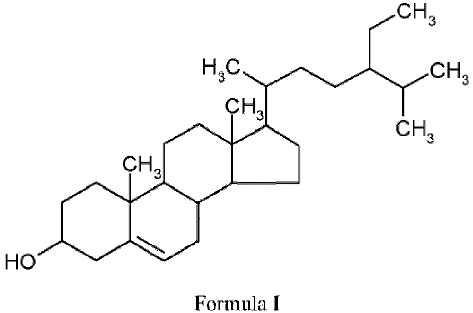

### β-Sitosterol, (3S,8S,9S,10R,13R,14S,17R)-17-((1R,4R)-4-ethyl-1,5-dimethyl-hexyl)-10,13-dimethyl-2,3,4,7,8,9,10,11,12,13,14,15,16,17-tetradecahydro-1H-cyclopenta[a]phenanthren-3-ol:

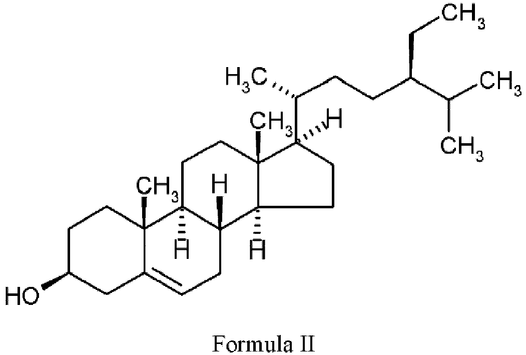

### Stigmasterol, (3S,8S,9S,10R,13R,14S,17R)-17-((E)-(1R,4S)-4-ethyl-1,5-dimethyl-hex-2-enyl)-10,13-dimethyl-2,3,4,7,8,9,10,11,12,13,14,15,16,17-tetradecahydro-1H-cyclopenta[a]phenanthren-3-ol:

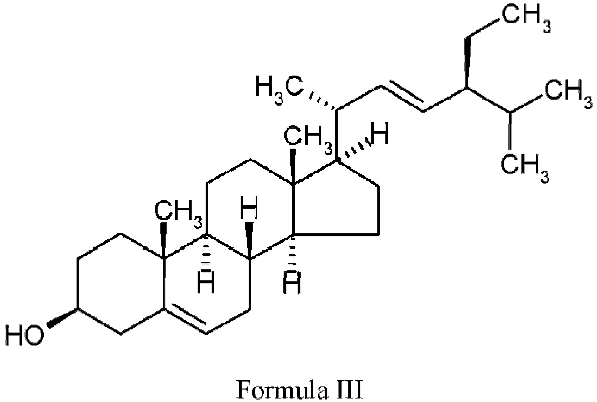

### Ergosterol, (3S,9S, 10R,13R,14R,17R)-10,13-dimethyl-17-((E)-(1R,4R)-1,4,5-trimethyl-hex-2-enyl)-2,3,4,9, 10,11,12,13,14,15,16,17-dodecahydro-1H-cyclopenta[a]phenanthren-3-ol:

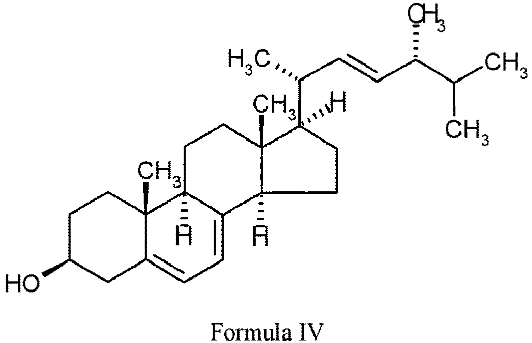

### Cholesterol, (3S,8S,9S,10R,13R,14S,17R)-17-((R)-1,5-dimethyl-hexyl)-10,13-dimethyl-2,3,4,7,8,9,10,11, 12,13,14,15,16,17-tetradecahydro-1H-cyclopenta[a]phenanthren-3-ol:

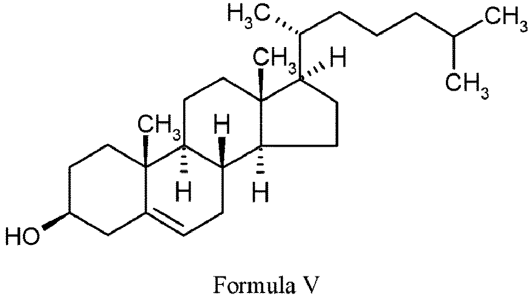

The sitosterol can be obtained commercially, synthesized according to procedures known in the art, for example, as described by Hang (Hang, et al. (2010) Steroids, 75(12): 879-883) or obtained from a variety of natural sources such as nuts, seeds, fruits, vegetables, plant oils and dark chocolate.

To date, there is no disclosure in the art of the flavor use associated with individual sterols, let alone the specific flavor enhancement alcohol sensation and carbonation effect that a particular sterol provides.

It has now been surprisingly discovered that sitosterol, 17-(4-ethyl-1,5-dimethyl-hexyl)-10,13-dimethyl-2,3,4,7,8,9,10,11,12,13,14,15,16,17-tetradecahydro-1H-cyclopenta[a]phenanthren-3-ol (Formula I), enhances alcohol sensation in alcoholic consumables or alcohol-free beverages. It has also been surprisingly found to enhance carbonation effect in carbonated beverages. Particularly, an isomer of sitosterol, β-sitosterol, (3S,8S,9S,10R,13R,14S,17R)-17-((1R,4R)-4-ethyl-1,5-dimethyl-hexyl)-10,13- dimethyl-2,3,4,7,8,9,10,11,12,13,14,15,16,17-tetradecahydro-1H-cyclopenta[a]phenanthren-3-ol (Formula II), is distinctly effective in enhancing alcohol sensation in alcoholic consumables or alcohol-free beverages. Further, β-sitosterol is distinctly effective in enhancing carbonation effect in carbonated beverages. Thus, sitosterol such as β-sitosterol provides superior ingredient performance and possesses unexpected advantages in specific flavor enhancement.

Accordingly, one embodiment of the present invention relates to the surprising finding of the unexpected effectiveness of sitosterol or an isomer thereof in enhancing alcohol sensation in an alcoholic consumable or an alcohol-free beverage. Another embodiment of the present invention relates to the particular effectiveness of β-sitosterol in enhancing alcohol sensation in an alcoholic consumable or an alcohol-free beverage. Another embodiment of the present invention relates to the surprising finding of the unexpected effectiveness of sitosterol or an isomer thereof in enhancing carbonation effect in a carbonated beverage. Another embodiment of the present invention relates to the particular effectiveness of β-sitosterol in enhancing carbonation effect in a carbonated beverage.

An alcoholic consumable is a food consumable that contains ethanol (CH₃CH₂OH), which includes, for example, an alcoholic beverage such as a distilled beverage, a wine, a beer or an alcohol-based fruit juice such as a cider, an alcoholic chocolate or an alcoholic candy. In certain embodiment, an alcoholic consumable contains ethanol in an amount greater than 0.1 percent by volume, preferably from 1 to 50 percent by volume and more preferably from 2 to 40 percent by volume.

The terms "alcohol-free beverage" and "non-alcoholic beverage" are understood to mean the same, which is a non-alcoholic version of the alcoholic beverage defined in the above such as an alcohol-free distilled beverage or an alcohol-free beer. An alcohol-free beverage or a non-alcoholic beverage provides alcohol impression as an alcoholic beverage does. In certain embodiment, an alcohol-free beverage contains ethanol in an amount of 0.0 percent by volume.

Alcohol sensation or alcohol impression refers to alcoholic mouthfeel that includes perceptions such as astringency, dryness, heating, hot, tingling, irritating, numbing, burning and/or cooling.

A carbonated beverage is a beverage incorporated with carbon dioxide (CO₂). A carbonated beverage includes, for example, a carbonated water, a mineral water, a soda, a beer, a sparkling wine or a Champagne. Carbon dioxide is dissolved in the beverage under pressure. Alternatively, or additionally, a carbonate salt can be dissolved in the beverage, wherein the carbonate salt contributes carbon dioxide. In certain embodiment, a carbonated beverage contains carbon dioxide of a concentration greater than 1 gram per liter (g L), preferably from 2 to 10 g/L and more preferably from 4 to 8 g/L.

The term "carbonate salt" is understood to mean a salt comprising a cation and a bicarbonate anion (HCO₃), a carbonate dianion (CO₃²⁻), or a combination thereof. A carbonate salt includes, for example, sodium bicarbonate (NaHCO₃), sodium carbonate (Na₂CO₃), potassium bicarbonate (KHCO₃), potassium carbonate (dipotassium carbonate, K₂CO₃), and a mixture thereof.

Carbonation effect refers to the pleasant, tingly sensation on the tongue created by the small bubbles of carbon dioxide released from a carbonated beverage. When pressure of a carbonated beverage is reduced, the dissolved carbon dioxide is expelled from the beverage as small bubbles. As a result, carbonic acid is formed in the oral mucosa and the small bubbles also cause tactile stimulation leading to carbonation effect including perceptions such as fizzy, tingling, burning and/or numbing.

The term "olfactory effective amount" is understood to mean the amount of sitosterol or an isomer thereof used in an alcoholic consumable, wherein sitosterol or an isomer thereof complements the alcohol sensation produced by ethanol, intensifies alcohol impression and therefore provides enhancement of alcohol sensation. In certain embodiment, the term "olfactory effective amount" is understood to mean the amount of β-sitosterol used in an alcoholic consumable. The term "olfactory effective amount" is also understood to mean the amount of sitosterol or an isomer thereof used in an alcohol-free beverage, wherein sitosterol or an isomer thereof increases the alcohol impression and therefore provides enhancement of alcohol sensation.. In certain embodiment, the term "olfactory effective amount" is understood to mean the amount of β -sitosterol used in an alcohol-free beverage. The term "olfactory effective amount" is also understood to mean the amount of sitosterol or an isomer thereof used in a carbonated beverage, wherein sitosterol or an isomer thereof intensifies the carbonation sensation produced by the carbonated beverage. In certain embodiment, the term "olfactory effective amount" is understood to mean the amount of β-sitosterol used in a carbonated beverage.

The olfactory effective amount may vary depending on many factors including other ingredients, their relative amounts and the olfactory effect that is desired. In the present invention, the olfactory effective amount of sitosterol or an isomer employed in an alcoholic consumable or an alcohol-free beverage is0.1 parts per billion or greater by weight, preferably ranges from 0.1 parts per billion to 5 parts per million by weight, more preferably from 1 part per billion to 1 part per million by weight and even more preferably from 10 to 250 parts per billion by weight. The term "ppm" is understood to mean part per million by weight. The term "ppb" is understood to mean part per billion by weight. In the present invention, the olfactory effective amount of sitosterol or an isomer employed in a carbonated beverage is0.1 parts per trillion or greater by weight, preferably ranges 0.1 to 10 parts per trillion by weight. The term "ppt" is understood to mean part per trillion by weight.

Additional materials can also be used in conjunction with the compound of the present invention to encapsulate and/or deliver the flavor enhancement effect. Some well-known materials are, for example, but not limited to, polymers, oligomers, other non-polymers such as surfactants, emulsifiers, lipids including fats, waxes and phospholipids, organic oils, mineral oils, petrolatum, natural oils, perfume fixatives, fibers, starches, sugars and solid surface materials such as zeolite and silica.

The invention is described in greater detail by the following non-limiting examples. Materials were purchased from Aldrich Chemical Company unless noted otherwise.

### Example I: Preparation of Test Samples

1. A series of β-sitosterol solutions with concentrations ranging from 2.5 to 600 ppb were prepared in water, β-sitosterol exhibited no noticeable flavor itself.
2. Alcohol solutions containing varying amounts of ethanol (Alcohol by Volume, "ABV") were prepared as follows:
   - Alcohol solutions at 1% ABV, 2% ABV, 2.5% ABV, 3% ABV and 5% ABV were prepared by adding varying amounts of ethanol in Brahma 0.0% alcohol-free beer (Anheuser-Busch InBev);
   - Alcohol solution at 6% ABV was prepared by adding ethanol in water; and
   - Alcohol solutions with ABV ranging from 8.75% to 50% were prepared by diluting CAPTAIN MORGAN^{®} Original Spiced Rum (35% ABV) (Diageo North America Inc.) in varying amounts of water.
3. Carbonated water containing carbon dioxide at 6.0, 7.2 and 8.0 g/L, respectively, were prepared.

### Example II: Enhancement of Alcohol Sensation in Alcohol-Free Beverages

A β-sitosterol solution (prepared in Example I) was added in Brahma 0.0% alcohol free beer to yield a final concentration of 25 ppb. The alcohol impression of the obtained sample was evaluated and compared by a panel with alcohol solutions at 1% ABV, 2% ABV, 2.5% ABV, 3% ABV and 5% ABV (prepared in Example I). β-Sitosterol (25 ppb) provided an equal level of alcohol impression as the alcohol solution at 2.5% ABV.

Accordingly, β-Sitosterol provided alcohol sensation in alcohol-free beverages.

### Example III: Enhancement of Alcohol Sensation in Alcoholic Beverages

### (A) A β-sitosterol solution (prepared in Example I) was added to the alcohol solution at 6% ABV (prepared in Example I) to yield a final concentration of 100 ppb.

The alcohol sensation of the obtained sample was evaluated and compared by a panel with the alcohol solution at 6% ABV using an intensity scale of 0 to 10, where 0 = none, 4 = medium, 7 = high and 10 = extremely high. The intensity of alcohol sensation was rated. Mean ("Alcohol Intensity") and standard error of the mean ("SE", ±) were obtained. The test results were reported in the following:

| **Sample** | **Alcohol Intensity** | **SE** |
|---|---|---|
| Solution of 6% ABV | 4.38 | 0.32 |
| Solution of 6% ABV + β-Sitosterol (100 ppb) | 7.21 | 0.27 |

The difference of alcohol intensity between the two groups was statistically significant (p < 0.01). Thus, β-sitosterol was effective in enhancing alcohol sensation in alcoholic beverages.

### (B) A series of β-sitosterol solutions (prepared in Example I) were added to the alcohol solution at 17.5% ABV (prepared in Example I) to yield final concentrations of β-sitosterol ranging from 3 to 500 ppb. The alcohol sensation of the obtained samples containing different amounts of β-sitosterol was evaluated.

At 3 ppb, β-sitosterol provided slight but noticeable increase in alcohol sensation. At a higher concentration, β-sitosterol produced clear increase of alcohol intensity. However, at 200 ppb, the enhancement of alcohol sensation of β-sitosterol reached a stable plateau.

### (C) A β-sitosterol solution (prepared in Example I) was added to alcohol solutions at 8.75% ABV, 17.5% ABV and 21.75% ABV (prepared in Example I), respectively, to yield a final concentration of 25 ppb. The alcohol sensation of the obtained samples was evaluated and compared by a panel with alcohol solutions at higher levels of ABV, respectively.

Alcohol solution at 8.75% ABV with added β-sitosterol was reported as having an alcohol sensation at the level of an alcohol solution at 10% ABV;
Alcohol solution at 17.5% ABV with added β-sitosterol was reported as having an alcohol sensation at the level of an alcohol solution at 22.5% ABV;
Alcohol solution at 21.75% ABV with added β -sitosterol was reported as having an alcohol sensation at the level of an alcohol solution at 35% ABV.

### Example IV: Enhancement of Carbonation Effect in Carbonated Beverages

### (A) A β -sitosterol solution (prepared in Example I) was added to the carbonated water (7.2 g/L CO₂) (prepared in Example I) to yield a final concentration of 2.5 ppt.

The carbonation effect of the obtained sample was evaluated and compared by a panel with the carbonated water using an intensity scale of 0 to 10, where 0 = none, 4 = medium, 7 = high and 10 = extremely high. The intensity of carbonation effect was rated. Mean ("Carbonation Intensity") and standard error of the mean ("SE", ±) were obtained. The test results were reported in the following:

| **Sample** | **Carbonation Intensity** | **SE** |
|---|---|---|
| Solution of 7.2 g/L CO₂ | 4.88 | 2.53 |
| Solution of 7.2 g/L, CO₂ + β-Sitosterol (2.5 ppt) | 7.19 | 1.73 |

The difference of carbonation intensity between the two groups was statistically significant (p 〈 0.01). Thus, β-sitosterol was effective in enhancing carbonation effect in carbonated beverages.

### (B) A series of β-sitosterol solutions (prepared in Example I) were added to the carbonated water (7.2 g/L CO₂) (prepared in Example I) to yield final concentrations ranging from 0.1 to 50 ppt. The carbonation sensation of the obtained samples containing different amounts of CO₂ was evaluated.

At 0.1 ppt, β-sitosterol provided slight increase in carbonation perception. At 2.5 ppt, β-sitosterol produced clear increase in carbonation perception. At a level of 5 ppt or higher, the enhancement of carbonation effect by β -sitosterol reached a stable plateau.

### (C) A β-sitosterol solution (prepared in Example I) was added to carbonated water of 6.0, 7.2 and 8.0 g/L CO₂ (prepared in Example I), respectively, to yield a final concentration of 2.5 ppt. The carbonation sensation of the obtained samples was evaluated.

In all samples containing different amounts of CO₂, β-sitosterol provided enhancement of carbonation sensation.

## Claims

1. A method of enhancing alcohol sensation in an alcoholic consumable or an alcohol-free beverage comprising the step of adding an olfactory effective amount of 17-(4-ethyl-1,5-dimethyl-hexyl)-10,13-dimethyl-2,3,4,7, 8,9,10,11,12,13,14,15,16,17-tetradecahydro-1H-cyclopenta[a]phenanthren-3-ol or an isomer thereof, wherein the olfactory effective amount is 0.1 parts per billion or greater by weight.

2. The method of claim 1, wherein the isomer is (3S,8S,9S,10R,13R,14S,17R)-17-((1R,4R)-4-ethyl-1,5-dimethyl-hexyl)-10,13-dimethyl-2,3,4,7,8,9,10,11,12,13,14,15,16,17-tetradecahydro-1H-cyclopenta[a]phenanthren-3-ol.

3. The method of claim 1, wherein the olfactory effective amount is from 0.1 parts per billion to 5 parts per million by weight.

4. The method of claim 1, wherein the olfactory effective amount is from 1 part per billion to 1 part per million by weight.

5. The method of claim 1, wherein the olfactory effective amount is from 10 to 250 parts per billion by weight.

6. The method of claim 1, wherein the alcoholic consumable is an alcoholic beverage selected from the group consisting of a distilled beverage, a wine, a beer and an alcohol-based fruit juice, or wherein the alcohol-free beverage is selected from the group consisting of an alcohol-free distilled beverage and an alcohol-free beer.

7. A method of enhancing carbonation effect in a carbonated beverage comprising the step of adding an olfactory effective amount of 17-(4-ethyl-1,5-dimethyl-hexyl)-10,13-dimethyl-2,3,4,7,8,9,10,11,12,13,14,15,16,17-tetradecahydro-1H-cyclopenta[a]phenanthren-3-ol or an isomer thereof, wherein the olfactory effective amount is 0.1 parts per trillion or greater by weight.

8. The method of claim 7, wherein the isomer is (3S,8S,9S,10R,13R,14S,17R)-17-((1R,4R)-4-ethyl-1,5-dimethyl-hexyl)-10,13-dimethyl-2,3,4,7,8,9,10,11,12,13,14,15,16,17-tetradecahydro-1H-cyclopenta[a]phenanthren-3-ol.

9. The method of claim 7, wherein the carbonated beverage is selected from the group consisting of a carbonated water, a mineral water, a soda, a beer, a sparkling wine and a Champagne.

## Patentansprüche

1. Verfahren zur Verstärkung der Alkoholempfindung in einem alkoholischen Verbrauchsgut oder einem alkoholfreien Getränk, umfassend den Schritt des Zugebens einer olfaktorisch wirksamen Menge von 17-(4-Ethyl-1,5-dimethylhexyl)-10,13-dimethyl-2,3,4,7,8,9,10,11,12,13,14,15,16,17-tetradecahydro-1H-cyclopenta[a]phenanthren-3-ol oder einem Isomer davon, wobei die olfaktorisch wirksame Menge, 0,1 Gewichtsteile pro Milliarde oder mehr beträgt.

2. Verfahren nach Anspruch 1, wobei es sich bei dem Isomer um (3S,8S,9S,10R,13R,14S,17R)-17-((1R,4R)-4-Ethyl-1,5-dimethylhexyl)-10,13-dimethyl-2,3,4,7,8,9,10,11,12,13,14,15,16,17-tetradecahydro-1H-cyclopenta[a]phenanthren-3-ol handelt.

3. Verfahren nach Anspruch 1, wobei die olfaktorisch wirksame Menge 0,1 Gewichtsteile pro Milliarde bis 5 Gewichtsteile pro Million beträgt.

4. Verfahren nach Anspruch 1, wobei die olfaktorisch wirksame Menge 1 Gewichtsteil pro Milliarde bis 1 Gewichtsteil pro Million beträgt.

5. Verfahren nach Anspruch 1, wobei die olfaktorisch wirksame Menge 10 bis 250 Gewichtsteile pro Milliarde beträgt.

6. Verfahren nach Anspruch 1, wobei es sich bei dem alkoholischen Verbrauchsgut um ein alkoholisches Getränk handelt, das aus der Gruppe bestehend aus einem destillierten Getränk, einem Wein, einem Bier und einem alkoholbasierten Fruchtsaft ausgewählt wird, oder wobei das alkoholfreie Getränk aus der Gruppe bestehend aus einem alkoholfreien destillierten Getränk und einem alkoholfreien Bier ausgewählt wird.

7. Verfahren zur Verstärkung des Kohlensäureeffekts in einem kohlensäurehaltigen Getränk, umfassend den Schritt des Zugebens einer olfaktorisch wirksamen Menge von 17-(4-Ethyl-1,5-dimethylhexyl)-10,13-dimethyl-2,3,4,7,8,9,10,11,12,13,14,15,16,17-tetradecahydro-1H-cyclopenta[a]phenanthren-3-ol oder einem Isomer davon, wobei die olfaktorisch wirksame Menge 0,1 Gewichtsteile pro Billion oder mehr beträgt.

8. Verfahren nach Anspruch 7, wobei es sich bei dem Isomer um (3S,8S,9S,10R,13R,14S,17R)-17-((1R,4R)-4-Ethyl-1,5-dimethylhexyl)-10,13-dimethyl-2,3,4,7,8,9,10,11,12,13,14,15,16,17-tetradecahydro-1H-cyclopenta[a]phenanthren-3-ol handelt.

9. Verfahren nach Anspruch 7, wobei das kohlensäurehaltige Getränk aus der Gruppe bestehend aus einem kohlensäurehaltigen Wasser, einem Mineralwasser, einer Soda, einem Bier, einem Schaumwein und einem Champagner ausgewählt wird.

## Revendications

1. Procédé d'augmentation de la sensation d'alcool dans un produit de consommation alcoolisé ou une boisson sans alcool comprenant l'étape d'ajout d'une quantité efficace sur le plan olfactif de 17-(4-éthyl-1,5-diméthyl-hexyl)-10,13-diméthyl-2,3,4,7,8,9,10,11,12,13,14,15,16,17-tétradécahydro-1H-cyclopenta[a]phénanthrène-3-ol ou d'un isomère correspondant, la quantité efficace sur le plan olfactif étant de 0,1 partie par milliard ou plus en poids.

2. Procédé selon la revendication 1, l'isomère étant (3S,8S,9S,10R,13R,14S,17R)-17-((1R,4R)-4-éthyl-1,5-diméthyl-hexyl)-10,13-diméthyl-2,3,4,7,8,9,10,11,12,13,14,15,16,17-tétradécahydro-1H-cyclopenta[a]phénanthrène-3-ol.

3. Procédé selon la revendication 1, la quantité efficace sur le plan olfactif étant de 0,1 partie par milliard à 5 parties par million en poids.

4. Procédé selon la revendication 1, la quantité efficace sur le plan olfactif étant de 1 partie par milliard à 1 partie par million en poids.

5. Procédé selon la revendication 1, la quantité efficace sur le plan olfactif étant de 10 à 250 parties par milliard en poids.

6. Procédé selon la revendication 1, le produit de consommation alcoolisé étant une boisson alcoolisée choisie dans le groupe constitué par une boisson distillée, un vin, une bière et un jus de fruits à base d'alcool, ou la boisson sans alcool étant choisie dans le groupe constitué par une boisson distillée sans alcool et une bière sans alcool.

7. Procédé d'augmentation de l'effet de carbonatation dans une boisson gazeuse comprenant l'étape d'ajout d'une quantité efficace sur le plan olfactif de 17-(4-éthyl-1,5-diméthyl-hexyl)-10,13-diméthyl-2,3,4,7,8,9,10,11,12,13,14,15,16,17-tétradécahydro-1H-cyclopenta[a]phénanthrène-3-ol ou d'un isomère correspondant, la quantité efficace sur le plan olfactif étant de 0,1 partie par trillion ou plus en poids.

8. Procédé selon la revendication 7, l'isomère étant (3S,8S,9S,10R,13R,14S,17R)-17-((1R,4R)-4-éthyl-1,5-diméthyl-hexyl)-10,13-diméthyl-2,3,4,7,8,9,10,11,12,13,14,15,16,17-tétradécahydro-1H-cyclopenta[a]phénanthrène-3-ol.

9. Procédé selon la revendication 7, la boisson gazeuse étant choisie dans le groupe constitué par une eau gazeuse, une eau minérale, un soda, une bière, un vin pétillant et un champagne.
